# EUROPEAN PATENT APPLICATION

(11) **EP 3 950 663 A1**
(43) Date of publication of application: **09.02.2022**
(21) Application number: 20189198.3
(22) Date of filing: 03.08.2020
(51) Int. Cl.: C07C 229/22, C07C 229/26, C07C 67/08, C07C 67/26

(54) **NEW METHOD FOR OBTAINING DIESTER COMPOUNDS USEFUL FOR THE MANUFACTURE OF NEW QUATERNARY AMMONIUM COMPOUNDS**

(71) Applicant: Rhodia Operations, 93300 Aubervilliers (FR)
(72) Inventor: BACK, Olivier, 69008 Lyon (FR)
(74) Representative: Ridray, Annabelle

(57) **Abstract**

The invention concerns a new, efficient and cost-effective method for obtaining diesters by reacting epoxides with carboxylic acids, typically through the formation of monohydroxyl-monoesters. The diesters can be used for the manufacture of new quaternary ammonium compounds with outstanding surfactant properties and improved biodegradability.

## Description

The present invention relates to a new method for obtaining diester compounds which can be used for the manufacture of new quaternary ammonium compounds, and to such new quaternary ammonium compounds.

Fatty quaternary ammonium compounds which have surfactant properties and can be used in respective applications have been described in the literature and are available commercially in a variety of different types from various suppliers.

WO 97/08284 discloses compositions comprising Guerbet alcohol betaine esters which are represented by the general formula

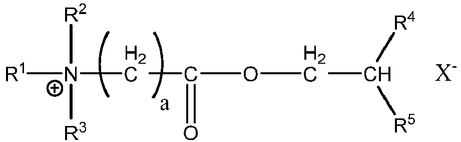

in which R¹ to R³ are independently selected from C₁ to C₄ alkyl groups or C₂-C₄ alkenyl groups, a is from 1 to 4 and R₄ and R₅ are independently selected from C₁₂ to C₂₂ alkyl or alkenyl groups, the sum of chain lengths of R₄ and R₅ preferably being at least 30.

EP 721 936 and DE 3402146 relate to quaternary ammonium compounds. As in WO 97/08284, the compounds comprise two long chain substituents which are esters of Guerbet acids.

While fatty quaternary ammonium compounds are widely used as surfactants, there is still a need for compounds of this type having a good combination of surfactant properties on one hand and biodegradability on the other hand. Biodegradability has become more and more important in the recent past due to the desire of customers to have more environmentally friendly products. The improvement in biodegradability should not negatively affect the surfactant properties.

It was thus an object of the present invention to provide new quaternary ammonium compounds with good surfactant properties and a good biodegradability.

This object is achieved with the compounds of formula (I). Preferred embodiments of the present invention are also detailed hereinafter.

The novel ionic compounds in accordance with the present invention have the general formula (I) 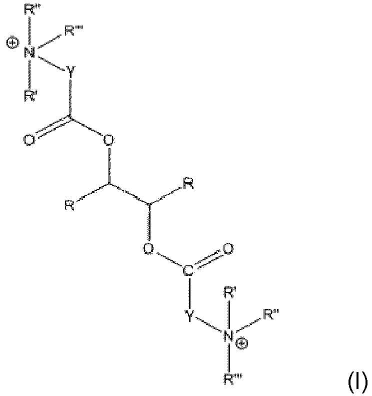 wherein
R, which may be the same or different at each occurrence, is a C₅-C₂₇ aliphatic group, preferably a C₆ to C₂₄ aliphatic group,
Y, which may be the same or different at each occurrence, is a divalent C₁-C₆ aliphatic radical,
R', R" and R'", which may be the same or different at each occurrence, are, independently from each other, hydrogen or a C₁ to C₄ alkyl group,

The aliphatic groups R may be free of any double bond and of any triple bond. Alternatively, the aliphatic groups R may comprise at least one -C=C- double bond and/or at least one -C=C- triple bond. The aliphatic groups R are advantageously chosen from alkyl groups, alkenyl groups, alkanedienyl groups, alkanetrienyl groups and alkynyl groups. The aliphatic groups R may be linear or branched. Preferably, the aliphatic groups R are independently chosen from alkyl and alkenyl groups. More preferably, the aliphatic groups R are independently chosen from alkyl and alkenyl groups, generally from C₆-C₂₄ alkyl and C₆-C₂₄ alkenyl groups, very often from C₆-C₂₁ alkyl and C₆-C₂₁ alkenyl groups and often from (i) C₆-C₁₉ alkyl and C₆-C₁₉ alkenyl groups or from (ii) C₆-C₁₇ alkyl and C₆-C₁₇ alkenyl groups. More preferably, R represent an alkyl group, generally a C₆-C₂₄ alkyl group, very often a C₆-C₂₁ alkyl group, often a C₆-C₁₉ alkyl group or a C₆-C₁₇ alkyl group. Aliphatic groups, in particular alkyl groups, with 10 to 20, preferably with 11 to 17 or with 10 to 17 carbon atoms have been found advantageous in certain cases. Acyclic aliphatic groups, more preferably linear aliphatic groups, still more preferably linear alkyl groups may be mentioned as preferred examples of substituents R. The number of carbon atoms of R can be even or odd and each group R can have the same number of carbon atoms or the number of carbon atoms of different groups R may be different.

R', R" and R'", which may be the same or different, are preferably hydrogen or a C₁ to C₄ alkyl group, preferably methyl or ethyl, more preferably methyl. Preferably at least one, more preferably at least two, more preferably all three of R', R" and R'" are a C₁ to C₄ alkyl group, preferably methyl or ethyl, most preferably methyl.

Y is preferably an acyclic divalent aliphatic group, more preferably a linear divalent aliphatic group, still more preferably a linear alkanediyl (alkylene) group and has 1 to 6, preferably 1 to 4 carbon atoms. Aliphatic group Y preferably has at least two carbon atoms, in particular 2 to 6 carbon atoms.

The compounds of formula (II) 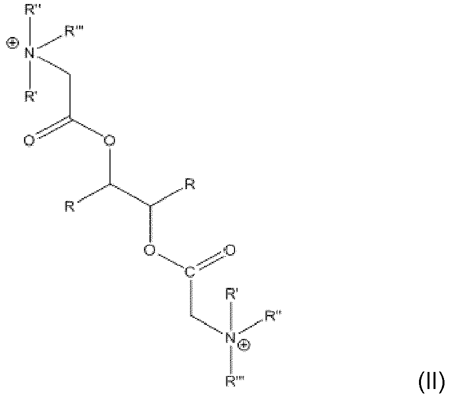 wherein R, R', R", R'" and Y in formula (II) have the meaning as defined for formula (I) as described hereinbefore represent particularly preferred compounds in accordance with the present invention.

The ionic compounds in accordance with the present invention can be obtained by a variety of methods. Preferred processes for the manufacture of the compounds of the present invention include the reaction of an internal ketone of formula R-C(=O)-R, which internal ketone may preferably be obtained by decarboxylative ketonization of a fatty acid, a fatty acid derivative or a mixture thereof. A suitable process for the manufacture of internal ketones following this route is diclosed in US 2018/0093936 to which reference is made for further details.

The synthesis of various compounds of the present invention using internal ketones obtainable as indicated above as starting materials is now described. The process variants described hereinafter show the synthesis of specific compounds and the skilled person will modify the reactants and reaction conditions based on his professional knowledge and taking into account the specific target product of the respective synthesis to manufacture other compounds in accordance with the present invention.

The compounds of formula (I) can preferably be obtained by two processes. The first process starts with a *Piria ketonization* followed by hydrogenation, dehydration, epoxydation (to obtain an epoxide), hydration (to obtain a diol) and esterification (to obtain a certain diester). This is a multi-step process plugged on Piria technology. It has the advantage of being salt-free and relying on chemical transformations which can be easily performed.

As an alternative to the above sequence of reactions, it is possible to bypass the hydration step (i.e. the formation of the diol) by converting straightforward the epoxide into the diester provided an appropriate esterification agent is used, as will be detailed later on.

The esterification step may be followed by an amine condensation step (as the final step) to convert the diester into a compound complying with formula (I).

Finally, starting from the diol, it also possible to obtain in one reaction step a compound which complies with formula (I), i.e. to achieve in one step said esterification and amine condensation, provided another appropriate esterification is used, as will be detailed later on.

### First process for synthesis of compounds represented by formula (I)

### Piria Ketonization

The basic reaction in the first step is:

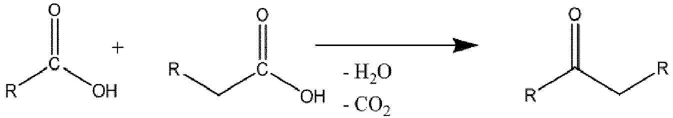

This reaction has been thoroughly described in US patent 10035746, WO 2018/087179 and WO 2018/033607 to which reference is made for further details.

### Hydrogenation

The internal ketone is then subjected to hydrogenation which can be carried out under standard conditions known to the skilled person for hydrogenation reactions:

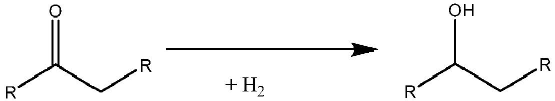

The hydrogenation reaction is conducted by contacting the internal ketone with hydrogen in an autoclave reactor at a temperature ranging from 15°C to 300°C and at a hydrogen pressure ranging from 1 bar to 100 bars. Details of this process step can e.g. be found in US patent 10035746 to which reference is made here.

### Dehydration

In the next step, the alcohol thus obtained is subjected to dehydration to obtain an internal olefin. This reaction can also be carried out under standard conditions known to the skilled person for respective dehydration reactions (e.g. US patent 10035746, example 4) so that no further details need to be given here:

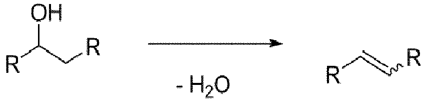

### Epoxidation

This internal olefin can thereafter be oxidized to the respective epoxide wherein the double bond is substituted by an epoxide group in accordance with the following scheme (where the reactants are just exemplary for respective groups of compounds serving the respective function): 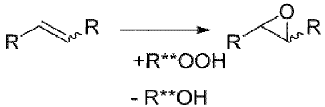 wherein R** can be hydrogen or a hydrocarbon group that can be substituted and/or interrupted by a heteroatom or heteroatom containing group, or R** can be an acyl group of general formula R***-C(=O)-wherein R*** can have the same meaning as R**.

The epoxide can be directly engaged in next step without further purification. This next step can be a hydration step (so as to form a diol, to be the followed by an esterification step) or a direct esterification step.

### Epoxide hydration (diol formation)

The epoxide can thereafter be hydrated to the respective diol in accordance with the following scheme:

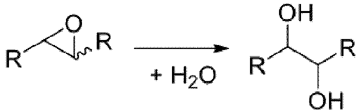

The ring opening reaction can be performed by contacting the epoxide with water, generally in the presence of a suitable catalyst and at a temperature ranging generally from 15°C to 150°C. As examples of catalysts one can mention Bronsted or Lewis acid catalysts.

### Esterification (starting from the diol)

The diol can be esterified according to the following reaction scheme: 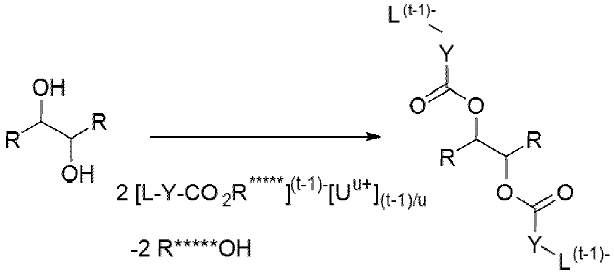 wherein
wherein R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
R***** is hydrogen or a C₁-C₆ alkyl group,
t is an integer which is equal to 1 or which is equal or superior to 2,
U^{u+} is a cation, and
u is an integer fixing the positive charge of the cation.

The esterification is first performed by contacting the diol with an esterification agent which is a carboxylic acid or an ester of a carboxylic acid of general formula:

[L-Y-CO₂R*****]^{(t-1)-}[U^{u+}]_{(t-1)/u}

wherein Y is a divalent hydrocarbon radical containing between 1 and 6 carbon atoms, more precisely a divalent C₁-C₆ aliphatic radical, and wherein L is a leaving group.

Y is preferably an acyclic divalent aliphatic group, more preferably a linear divalent aliphatic group, still more preferably a linear alkanediyl (alkylene) group. Y has preferably from 1 to 6, more preferably from 1 to 4 carbon atom(s), still more preferably 1 or 2 carbon atom(s). The most preferred Y is -CH₂-.

When t is equal to 1, no cation is present. Otherwise said, the esterification is performed by contacting the diol with a carboxylic acid or an ester of a carboxylic acid of formula:

L-Y-CO₂R*****.

In the case the leaving group L already carries a negative charge in the carboxylic acid or ester reactant (this is the case when (t-1) is equal or superior to 1 or when t is equal or superior to 2), a cation noted U^{u+} (with u preferably being 1, 2 or 3, even more preferably 1) must be present in the reactant to ensure the electroneutrality (in this case the cation possesses a u⁺ charge). This cation may e.g. be selected from H⁺, alkaline metal cations, alkaline earth metal cations (e.g. Na⁺, K⁺, Ca²⁺), Al³⁺ and ammonium, to mention only a few examples.

The nature of the leaving group L is not particularly limited provided next reaction step (i.e. amine condensation, as will be detailed later on) can occur. The leaving group L is preferably chosen from:
- a halogen, such as fluorine, chlorine, bromine or iodine,
- a (hydrocarbyloxysulfonyl)oxy group of formula R^{a}-O-SO₂-O- wherein R^{a} denotes a C₁-C₂₀ hydrocarbyl group, such as CH₃-O-SO₂-O-, and
- an oxysulfonyloxy group of formula -O-SO₂-O-.

An example for a compound with t equal to 1 is the compound CH₃-O-SO₃-CH₂-COOR***** which, for R***** being H, yields the compound CH₃-O-SO₃-CH₂-COOH which can be designated as 2-((methoxysulfonyl)oxy)acetic acid.

As further examples of compounds in which t is equal to 1 and thus no cation is present, one can mention: chloroacetic acid, bromoacetic acid and 2-chloropropionic acid.

An example for t being equal to 2 is sodium carboxymethylsulfate acid in which [L-Y-COOR*****]^{(t-1)-}[U^{u+}]_{(t-1)/u} is [Na⁺] [O-SO₂-O-CH₂-COOR*****]⁻ with R***** being H, U being Na and thus [U^{u+}]_{t/u}[L^{t-}] being Na₂SO₄ The esterification can preferably be conducted at a temperature ranging from 50°C to 250°C in the presence of an optional solvent. However the presence of such solvent is not mandatory and the reaction can be also conducted without any added solvent. As example of suitable solvents one can mention: toluene, xylene, hydrocarbons, DMSO, Me-THF, THF or mixtures thereof.

Water that is formed as a by-product during the reaction can be removed from the reaction medium by distillation over the course of the reaction.

A catalyst can also be employed during the reaction and suitable catalysts are Bronsted or Lewis acid catalysts. As preferred examples of catalysts one can mention: H₂SO₄, *para*-toluenesulfonic acid, trifluoromethanesulfonic acid (commonly referred to as triflic acid), HCl, or heterogeneous acidic resins such as Amberlite^{®} resins, AlCl₃ etc. At the end of the reaction, the desired diester can be recovered after appropriate work-up and the skilled person is aware of representative techniques so that no further details need to be given here.

As will be seen later on, the diester of formula (III) 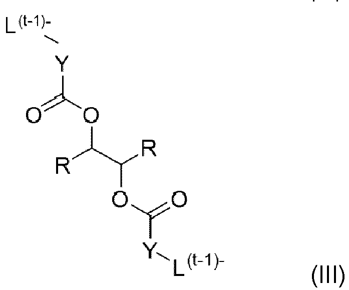 can be easily converted into a compound of formula (I) as previously represented, which exhibits outstanding surfactant properties.

### Direct esterification (starting from the epoxide, including the epoxide ring-opening)

The Applicant has surprisingly found that the epoxide can be directly esterified into the diester of formula (III) when and only when a carboxylic acid is used as the esterification agent [L-Y-CO₂R*****]^{(t-1)-}[U^{u+}]_{(t-1)/u}, that is to say when the esterification agent [L-Y-CO₂R*****]^{(t-1)-}[U^{u+}]_{(t-1)/u} is of the formula

[L-Y-CO₂H]^{(t-1)-}[U^{u+}]_{(t-1)/u},

wherein L, Y, t, U^{u+} and u are as described here before in connection with the esterification of the diol.

The epoxide ring-opening and esterification take then place according to the following reaction scheme: 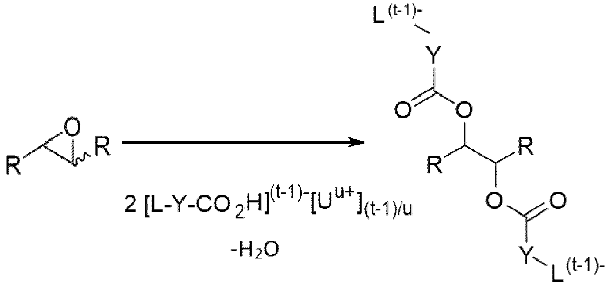 wherein
R, which may be the same or different at each occurrence, is a C₅-C₂₇ aliphatic group, preferably a C₆ to C₂₄ aliphatic group,
L is a leaving group,
Y is a divalent C₁-C₆ aliphatic radical,
t is an integer which is equal to 1 or which is equal or superior to 2,
U^{u+} is a cation, and
u is an integer fixing the positive charge of the cation.

The epoxide ring-opening and esterification are performed by reacting the epoxide with a carboxylic acid of general formula:

[L-Y-CO₂H]^{(t-1)-}[U^{u+}]_{(t-1)/u}

wherein L is a leaving group, Y is a divalent C₁-C₆ aliphatic radical, t is an integer which is equal to 1 or which is equal or superior to 2, U^{u+} is a cation, and u is an integer fixing the positive charge of the cation.

All the details which have been provided here before concerning L, Y, t, U^{u+} and U in connection with the esterification of the diol are also valid and can apply here in connection with the direct esterification of the epoxide, so that such details need not to be repeated.

In particular, when t is equal to 1, no cation is present. Otherwise said, the esterification is performed by contacting the epoxide with a carboxylic acid of formula:

L-Y-CO₂H.

To the Applicant's knowledge, the direct esterification of an epoxide bearing 2 long aliphatic chains, such as the epoxide of formula 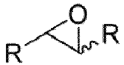 wherein each R, which may be the same or different at each occurrence, is a C₅-C₂₇ aliphatic group, is new.

In Ber. Bunsenges. Phys. Chem., 100, 1335-1340 (1996), No. 8, it can be found a thermokinetics study of the reaction of bromomethyloxirane with dichloroacetic acid. The moeities of bromomethyloxirane which "correspond" to the R of above formula are respectively H and CH₂Br, and the moeity of dichloroacetic acid which "corresponds" to Y in the formula of the esterifying agent is -CHCI-. None of such "R-equivalent" and "Y-equivalent" moieties is an aliphatic group.

Garcia et al., in Journal of Industrial Microbiology, 28, 173-179 (2002), 173-179, realized the enzymatic esterification of 1,2-epoxy-5-hexene with 2-chlorobutyric acid in the presence of an immobilized lipase from *Mucor miehei* to obtain 2-chloroesters. The moeities of 1,2-epoxy-5-hexene which "correspond" to the R of above formula are H and -(CH₂)₂-CH=CH₂. Garcia did not consider the enzymatic esterification of an epoxide the "R-equivalent" moieities of which would be both aliphatic. A fortiori, Garcia did not consider the direct epoxidation of an epoxide the "R-equivalent" moieties of which would be both aliphatic in the absence of an enzyme as catalyst.

More generally, none of these background art citations, which all related to the esterification of an epoxide having one and only one aliphatic or halogenoaliphatic group, describes or suggests the esterification of an epoxide of formula 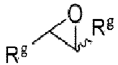 wherein each R^{g}, which may be the same or different at each occurrence, would be an aliphatic or halogenophatic group with a carboxylic acid of general formula [L-Y-CO₂H]^{(t-1)-}[U^{u+}]_{(t-1)/u}, wherein L, Y, t, U^{u+} and u are as described here before.

It was thus another object of the present invention to provide a new, efficient and cost-effective method for the esterification of such optionally halogenated dialiphatic epoxides of formula 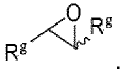 In particular, it was another object of the present invention to a provide a new, efficient and cost-effective method for the obtention of diesters from long chain dialiphatic epoxides of formula 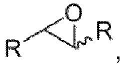 wherein such diesters can be advantageously used for the manufacture of new quaternary ammonium compounds with good surfactant properties and a good biodegradability.

This other object is achieved with a method which is described hereinafter under item 1:
- item 1 : a method for obtaining a diester having the general formula (IV) 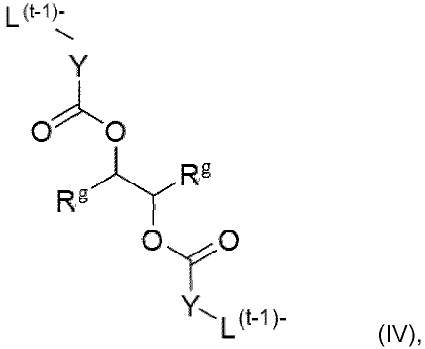 said method comprising reacting an epoxide of formula (V) 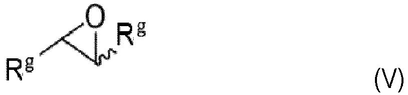 with a carboxylic acid of general formula (VI)

   [L-Y-CO₂H]^{(t-1)-}[U^{u+}]_{(t-1)/u} (VI)

   wherein, wherever used in above formulae :
   - R^{g}, which may be the same or different at each occurrence, is an aliphatic or halogenophatic group ;
   - Y is a divalent C₁-C₆ aliphatic radical ;
   - L is a leaving group ;
   - t is an integer which is equal to 1 or which is equal or superior to 2 ;
   - U^{u+} is a cation, and
   - u is an integer fixing the positive charge of the cation (e.g. 1 for H⁺, NH₄⁺ or an alkaline metal cation or 2 for an alkaline earth metal cation).
      It is understood that, likewise R^{g}, Y and L^{(t-1)-} may be the same or different at each occurrence.

Preferred and/or particular embodiments related to this invented method are set hereinafter:
- item 2: the method as described in item 1 wherein the reaction proceeds with the elimination of one mole of water by one mole of epoxide, as can be represented by: 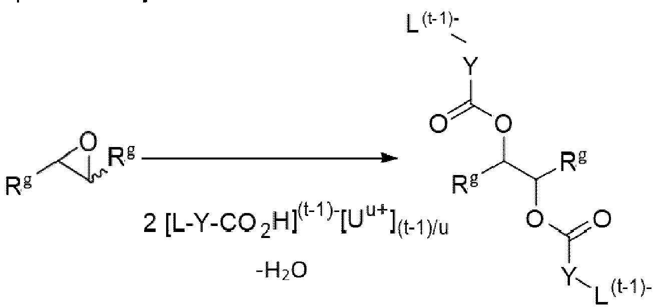
- item 3: the method as described in item 1 or 2 wherein the reaction takes place in a reaction medium from which water is removed by distillation over the course of the reaction ;
- item 4: the method as described in item 1, 2 or 3 wherein the reaction is conducted in the absence of enzyme ;
- item 5: the method as described in any one of items 1 to 4 wherein the reaction is conducted under an inert atmosphere, such as a nitrogen or rare gas atomosphere ;
- item 6: the method as described in item 5 wherein the inert atmosphere is an argon atmosphere ;
- item 7: the method as described in any one of items 1 to 6 wherein the total number of moles of carboxylic acid of formula (VI) which is contacted with the epoxide during the whole course of the reaction exceeds the number of moles of epoxide ;
- item 8: the method as described in item 7 wherein the total number of moles of carboxylic acid of formula (VI) which is contacted with the epoxide during the whole course of the reaction is at least twice higher than the number of moles of epoxide ;
- item 9: the method as described in item 8 wherein the total number of moles of carboxylic acid of formula (VI) which is contacted with the epoxide during the whole course of the reaction is at least four times higher than the number of moles of epoxide ;
- item 10: the method as described in any one of items 1 to 9 wherein the total number of moles of carboxylic acid of formula (VI) which is contacted with the epoxide during the whole course of the reaction is at most ten times higher than the number of moles of epoxide ;
- item 11: the method as described in item 10 wherein the total number of moles of carboxylic acid of formula (VI) which is contacted with the epoxide during the whole course of the reaction is at most eight times higher than the number of moles of epoxide ;
- item 12: the method as described in any one of items 1 to 11 wherein the reaction takes place in a reactor where the epoxide is in molten state ;
- item 13: the method as described in any one of items 1 to 12 wherein the reaction takes place in a reactor where the carboxylic acid of formula (VI) is in molten state ;
- item 14: the method as described in any one of items 1 to 13 wherein the epoxide is added progressively in a reactor containing the whole amount of the carboxylic acid of formula (VI) ;
- item 15: the method as described in item 14 wherein the epoxide is added continuously in a reactor containing the whole amount of the carboxylic acid of formula (VI) ;
   the Applicant has observed that contacting progressively, preferably continuously, the epoxide with the whole amount of the carboxylic acid made it possible to limit the self condensation of the epoxide ;
- item 16: the method as described in any one of items 1 to 15 which comprises:
   - a first step S₁ wherein the epoxide is reacted with the carboxylic acid of formula (VI) at a temperature T₁ below 100°C for a time t₁ which is sufficient to convert more than f₁=80 mol.% of the epoxide into a monohydroxyl-monoester of formula (VII): 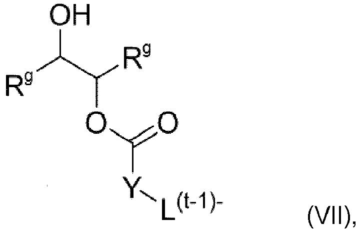
   - a second step S₂ wherein the monohydroxyl-monoester and the epoxide which has not been converted at step S₁ into the monohydroxyl-monoester are reacted with the carboxylic acid of formula (VI) at a temperature T₂ of at least 100°C for a time t₂ which is sufficient to form the diester of formula (IV) in a molar amount greater than f₂=50 mol.% of the total amount of epoxide which is reacted with the carboxylic acid;
      the Applicant has surprisingly found that reacting firstly the epoxide with the carboxylic acid at a temperature T₁ below 100°C for a time t₁ sufficient to convert more than f₁=80 mol.% of the epoxide into a monohydroxyl-monoester (step S₁) before increasing the temperature to convert the monohydroxyl-monoester into the diester (step S₂) made it possible to limit the formation of ketone and dehydration by-products ;
- item 17: the method as described in item 16 wherein the whole amount of the epoxide is added progressively during step S₁ in a reactor containing the whole amount of the carboxylic acid of formula (VI) ;
- item 18: the method as described in item 17 wherein the whole amount of the epoxide is added continuously during step S₁ in a reactor containing the whole amount of the carboxylic acid of formula (VI) ;
- item 19: the method as described in any one of items 16 to 18 wherein T₁ is of at most 80°C ;
- item 20: the method as described in item 19 wherein T₁ is of at most 70°C ;
- item 21: the method as described in any one of items 16 to 20 wherein T₁ is of at least 25°C ;
- item 22: the method as described in item 21 wherein T₁ is of at least 40°C, preferably at least 55°C, more preferably at least 60°C ;
- item 23: the method as described in any one of items 16 to 22 wherein f₁=90 mol.% ;
- item 24: the method as described in item 23 wherein f₁=95 mol.% ;
- item 25: the method as described in item 24 wherein f₁=98 mol.% ;
- item 26: the method as described in any one of items 16 to 25 wherein t₁ ranges from 10 min to 10 h ;
- item 27: the method as described in item 26 wherein t₁ is of at least 30 min, possibly at least 45 min ;
- item 28: the method as described in item 26 or 27 wherein t₁ is of at most 4 h, preferably from 30 min to 3 h;
- item 29: the method as described in any one of items 16 to 28 wherein T₂ is of at least 120°C ;
- item 30: the method as described in item 29 wherein T₂ is of at least 130°C ;
- item 31: the method as described in any one of items 16 to 30 wherein T₂ is of at most 250°C,
- item 32: the method as described in item 31 wherein T₂ is of at most 200°C, preferably at most 170°C, more preferably at most 150°C ;
- item 33: the method as described in any one of items 16 to 32 wherein the diester of formula (IV) is formed in a molar amount greater than f₂=65 mol. % ;
- item 34: the method as described in item 33 wherein f₂=70 mol.% ;
- item 35: the method as described in item 34 wherein f₂=75 mol.% ; preferably, the diester of formula (IV) is formed in a molar f₂ of at least 80 mol. % ;
- item 36: the method as described in any one of items 16 to 35 wherein t₂ ranges from 1 h to 30 h ;
- item 37: the method as described in item 36 wherein t₂ ≥ 3 h ;
- item 38: the method as described in item 37 wherein t₂ ≥ 4h ;
- item 39: the method as described in item 36, 37 or 38 wherein t₂ is of at most 10 h ;
- item 40: the method as described in item 39 wherein t₂ ≤ 7 h ;
- item 41: the method as described in any one of items 16 to 40 wherein step S₂ is conducted at a pressure P₂ of at most 90 kPa, preferably from 50 kPa to 90 kPa, more preferably from 70 kPa to 90 kPa, e.g. about 80 kPa ;
- item 42: the method as described in any one of items 16 to 40 wherein step S₂ is conducted at a pressure P₂ from 95 kPa to 99 kPa, preferably from 96 kPa to 98 kPa, e.g. about 97.5 kPa ;
   the Applicant has observed that conducting step S₂ under a light vacuum made it easier to remove the water formed as by-product of the esterification ;
- item 43: the method as described in item 41 or 42 which comprises, upon completion of step S₂, reducing progressively the pressure P₂ to a pressure P₃ below 100 kPa, preferably of at most 50 kPa, more preferably of at most 20 kPa, e.g. about 10 kPa ;
   the Applicant has observed that this made it possible to distill the excess of the carboxylic acid and to boost the conversion from the monohydroxyl-monoester into the diester;
- item 44: : the method as described in any one of items 1 to 43 wherein the reaction is conducted in the presence of a Bronsted or Lewis acid catalyst;
- item 45 : the method as described in any one of items 1 to 43 wherein the reaction is conducted in the presence of a catalyst chosen from H₂SO₄, *para*-toluenesulfonic acid, trifluoromethanesulfonic acid, HCl, AlCl₃ and a heterogeneous acidic resin such as an Amberlite^{®} resin ;
- item 46 : the method as described in any one of items 1 to 43 wherein the reaction is conducted in the absence of any catalyst;
- item 47 : the method as described in any one of items 1 to 46 wherein the reaction is conducted in the absence of solvent;
- item 48 : the method as described in any one of items 1 to 46 wherein the reaction is conducted in the presence of a solvent, such as toluene, xylene, a hydrocarbon, DMSO, Me-THF or THF ;
- item 49: the method as described in any one of items 1 to 48 wherein R^{g} (i.e. each R^{g}) is an aliphatic group ;
- item 50: the method as described in any one of items 1 to 49 wherein R^{g} comprises at least 2, possibly at least 3 or at least 4 carbon atoms ;
- item 51: the method as described in any one of items 1 to 50 wherein R^{g} comprises up to 4 carbon atoms ;
- item 52: the method as described in any one of items 1 to 50 wherein R^{g} comprises at least 5 carbon atoms ;
- item 53: the method as described in any one of items 1 to 48 wherein R^{g} (i.e. each R^{g}), which may be the same or different at each occurrence, represents a C₅-C₂₇ aliphatic group, i.e. R^{g} has the same definition as R, as previously described ;
- item 54: the method as described in item 53 wherein the obtained diester of formula (IV) complies with any one of the preferred or particular features of the diester of formula (III), notably concerning R^{g}=R, Y, L and t ;
- item 55: the method as described in item 53 or 54 which comprises reacting an olefin of formula 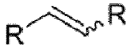 with an oxidizing agent of formula R**OOH with R** as described in the part of the description relative to the epoxidation of an olefin, entitled "Epoxidation" ;
- item 56: the method as described in item 55 which comprises obtaining the olefin of formula 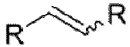 by applying a reaction scheme comprising a Piria ketonization step, a hydrogenation step and a dehydration step, as previously described ;
- item 57: the method as described in any one of items 1 to 56 wherein U^{u+} is chosen from H⁺, NH₄⁺, alkaline metal cations, alkaline earth metal cations and Al⁺³ ;
- item 58: a monohydroxyl-monoester compound of formula (VII) : 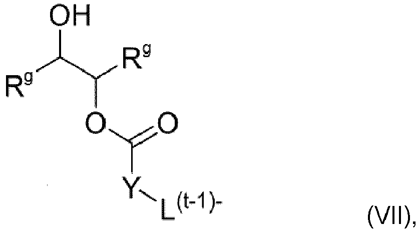 wherein
   - R^{g}, which may be the same or different at each occurrence, is an aliphatic or halogenophatic group,
   - Y is a divalent C₁-C₆ aliphatic radical,
   - L is a leaving group, and
   - t is an integer which is equal to 1 or which is equal or superior to 2 ;
- item 59: the monohydroxyl-monoester compound as described in item 58 wherein R^{g} (i.e. each R^{g}), which may be the same or different at each occurrence, represents a C₅-C₂₇ aliphatic group, i.e. R^{g} has the same definition as R, as previously described ;
- item 60: the monohydroxyl-monoester compound as described in item 59 wherein R, Y, L and t comply with one or more feature(s) described in connection with the diester of formula (III) ;
- item 61: use of the monohydroxyl-monoester compound as described in item 58, 59 or 60 for the manufacture of a diester compound ;
- item 62: use as described in item 61 wherein the monohydroxyl-monoester compound is as described in item 59 or 60.

### Amine condensation from the diester

The diester compound of formula (III) can be converted into the ionic compound of formula (I) through the following reaction scheme: 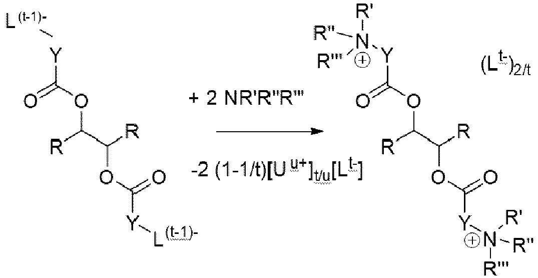 wherein
- R, Y, L, t, U^{u+} and U are as described here before, and
- R', R" and R'" are C₁ to C₄ alkyl groups, preferably methyl or ethyl, most preferably methyl.

The amine condensation reaction is performed by contacting the intermediate diester obtained as described above with an amine of general formula NR'R"R'" where R', R" and R'" are C₁ to C₄ alkyl groups, preferably methyl or ethyl, most preferably methyl.

The reaction can be conducted at a temperature ranging from 15°C to 250°C in the presence of a suitable solvent. As example of a suitable solvent one can mention: THF, Me-THF, methanol, ethanol, isopropanol, DMSO, toluene, xylene or their mixture. Alternatively the reaction can be also conducted in the absence of any added solvent.

During this reaction, there is a nucleophilic attack of the amine that substitues L^{(t-1)-} in the diester, L^{(t-1)-} plays the role of the leaving group. L^{t-} becomes then the counter-anion of the final quaternary ammonium compound. In the case the leaving group already carries a negative charge in the diester reactant (this is the case when (t-1) is equal or superior to 1 or when t is equal or superior to 2) there is also formation of a salt as the by-product of the reaction (with the general chemical formula [U^{u+}]_{t/u}[L^{t-}] as shown in the equation scheme above).

### Esterification and amine condensation / direct quaternization from the diol

Starting from the diol of formula 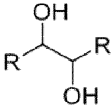 wherein R is as described here before, the Applicant has found that it is possible to obtain in one reaction step a compound which complies with formula (I), i.e. to achieve in one step said esterification and amine condensation, provided another appropriate esterification agent is used.

This appropriate esterification agent can be generally defined as an activated form of an amino-acid or an ester thereof of formula

R'R"R"' ⁺N-Y-C(=O)-O⁻

or their hydrohalogenide adducts of formula

R'R"R"' ⁺N-Y-C(=O)-OH X-

wherein
- R', R" and R'", which may be the same or different, are hydrogen or a C₁ to C₄ alkyl group,
- Y is a divalent C₁-C₆ aliphatic radical, and
- X denotes a halogen atom, such as chlorine or bromine.

The activated form of the amino-acid or ester thereof or their hydrohalogenide adducts is advantageously an acyl halogenide of formula

R'R"R"' ⁺N-Y-C(=O)-X X⁻

wherein R', R", R'", Y and X are as described before ; in particular, it can be an acyl chloride of formula R'R"R"' ⁺N-Y-C(=O)-Cl Cl⁻.

### Second process for synthesis of compounds represented by formula (I)

### Acyloin condensation

An alternative process for the synthesis of compounds of formula (I) proceeds via an acyloin condensation in accordance with the following scheme: 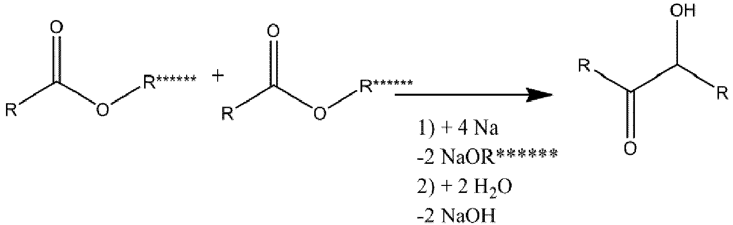 wherein R****** is an alkyl group having from 1 to 6 carbon atoms.

### Keto-alcohol hydrogenation

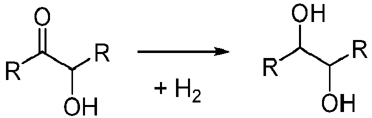

The subsequent reaction steps are as described hereinbefore for the first process variant.

The compounds of formula (I) are advantageously used as surfactants. They possess indeed a particularly interesting and advantageous property profile of surfactant properties on one hand and biodegradability properties on the other hand. Biodegradability is becoming more and more an important aspect for surfactant products.

The adsorption of a cationic surfactant on negatively charged surfaces is also an important property for such surfactants. This property is usually linked to the minimum concentration of surfactant needed to produce aggregation of a negatively charged cellulose nanocrystal (CNC, which is often used as reference material)) suspension in aqueous media. Consecutive variation of size can be monitored and followed by dynamic light scattering (DLS). Following the protocol described in E.K. Oikonomou et al., J. Phys. Chem. B, 2017, 121 (10), 2299-307 the adsorption properties of the quaternary ammonium compounds can be investigated by monitoring the ratio X=[surfactant]/[CNC] or the mass fraction M=[surfactant]/([surfactant + [CNC]), at fixed [surfactant] + [CNC] = 0.01 wt% in aqueous solution, required to induce the agglomeration of the cellulose nanocrystals.

The biodegradability of the compounds of the present invention can be determined in accordance with procedures described in the prior art and known to the skilled person. Details about one such method, OECD standard 301, are given in the experimental section hereinafter.

### Working examples

### Example 1 - First synthesis of a quaternary di-ammonium compound starting from 16-hentriacontanone

16-hentriacontanone was purchased from TCI, but could have been obtained from palmitic acid following Piria ketonization, for example in accordance with the protocol described in US patent 10035746, chain of examples 2 to 4.

### Hydrogenation of 16-hentriacontanone to 16-hentriacontanol

In a 100 mL autoclave equipped with a mechanical stirrer (Rushton turbine) were added:
- 4.36 g of Ru/C (4.87% Ru) catalyst (5 wt% of dry catalyst with respect to the ketone, catalyst containing 54.9% H₂O)
- 39.3 g (87.2 mmol) of melted C₃₁ ketone.

The reaction was performed under 20 bar hydrogen pressure. 4 nitrogen purges are performed followed by 3 purges of hydrogen at 20 bars. The temperature of the reaction mixture was then set at 100°C to melt the ketone substrate. The temperature was left at 100°C during 10 min and stirring was slowly started at 200 rpm. When proper stirring was confirmed, the stirring rate was increased at 1200 rpm and the temperature was set at 150°C.

After 6h reaction time at 150°C, heating was stopped and the mixture was allowed to cool down at 90°C while stirring. Stirring was then stopped. The mixture was cooled down to room temperature and the autoclave was carefully depressurized.

NMR analysis in CDCl₃ of the crude showed a ketone conversion level >99% and molar purity of 99% for the fatty alcohol. The compact solid containing the product and the catalyst was grounded to powder and then introduced into a 1L flask. 500 mL of chloroform were added and the flask was then heated at 60°C to dissolve completely the alcohol. The suspension was filtered at 60°C over celite. The solid cake was rinsed with hot chloroform at 60°C several times. The filtrate was evaporated to give 35.6 g (78.7 mmol) of white powder with a weight purity around 99% for the desired C₃₁ fatty alcohol corresponding to 90% isolated yield.

### Dehydration of 16-hentriacontanol to internal olefin

The reaction was conducted under an inert argon atmosphere. In a 200 mL quartz reactor equipped with a heating mattress, a mechanical stirrer (A320-type stirring mobile manufactured by 3D-printing with Inox SS316L), a condenser connected to a 50 mL two-neck distillate collection flask and a temperature probe were added:
- 56.1 g of C₃₁ fatty alcohol (124 mmol, 1 eq.)
- 5.61 g (10 wt%) of Al₂O₃-η.

The temperature of the reaction media was then increased to 150°C to melt the alcohol and stirring was started (about 500 rpm). Finally the temperature was set-up at 300°C and the mixture was allowed to stir at 1000 rpm under argon. Reaction progress was monitored thanks to NMR analysis with a borosilicate glass tube.

After 2 hours reaction at 300°C, NMR analysis in CDCl₃ showed complete conversion of the fatty alcohol and the presence of 1.5 mol% ketone which had been formed as a by-product.

Stirring and heating were then stopped. The temperature was lowered to 80°C and the molten crude was transferred to a beaker. The reactor vessel and the stirring mobile were rinsed with chloroform (Al₂O₃ is insoluble). The suspension was filtered and the solvent was evaporated under vacuum to afford 52.24 g (120.2 mmol) of transparent oil which became a white solid (99 wt% purity) at room temperature corresponding to 97% isolated yield (NMR).

### Epoxidation of internal olefin to oxirane

The reaction was conducted under an inert argon atmosphere. In a double-jacketed 1L reactor equipped with a mechanical stirrer (propeller with four inclined plows), a condenser and a temperature probe were added 92.4 g of C₃₁ alkene (0.212 mol), followed by 18.2 mL (19.1 g, 0.319 mol) of acetic acid and 27.7 g (30 wt%) of Amberlite^{®} IR 120H resin.

The mixture was heated to 75°C in order to melt the fatty alkene. The agitation was then started and 32.6 mL (36.1 g, 0.319 mol) of aqueous H₂O₂ 30% were slowly added into the mixture using an addition funnel while reaction mass temperature was monitored to avoid temperature increase. The addition required about one hour.

After the end of H₂O₂ addition, the temperature was increased to 85°C and reaction progress was followed thanks to NMR analysis. After 4 hours reaction time, the olefin conversion level was >99% with around 99% selectivity toward the desired epoxide (trace amounts of valuable diol were also formed during the reaction).

Heating was then stopped and 300 mL of chloroform were added to the reaction vessel when the temperature was around 50°C. The mixture was transferred to a separating funnel. The organic phase was washed 3 times with 300 mL of water. Then, the aqueous phase was extracted twice with 100 mL of chloroform. Amberlite^{®} resin was removed during phase separation with the aqueous phase. The organic phase was dried over MgSO₄, filtered and evaporated to give 95.3 g of a white solid with a purity of 98% w/w (epoxide + diol). The yield taking into account the purity was 97%.

### Condensation with chloroacetic acid (with H₂SO₄ as an optional catalyst)

The reaction was conducted under an inert argon atmosphere in a 500 mL three necked round bottom flask equipped with a magnetic stirrer, a heater, a condenser, a temperature probe and an insulated addition funnel. In the round bottom flask itself were added 128.7 g of chloroacetic acid (1.35 moles, 8 eq). In the insulated addition funnel maintained at 65°C were added 77.8 g of melted fatty epoxide (98 wt% purity, 0.169 moles, 1 eq).

The first step of intermediate hydroxyl-ester formation was conducted at 65°C by the slow addition of the fatty epoxide into chloroacetic acid in order to limit the formation of ketone and epoxide self-condensation by-products. The fatty epoxide was therefore added drop-wise over 30 minutes into the reactor containing chloroacetic acid at 65°C under stirring.

At the end of the addition, the mixture was allowed to stir at 65°C during additional 20 min. NMR analysis showed a conversion level >99 % for the starting epoxide.

Then, for the formation of the final bis-ester, 0.19 mL of 95% H₂SO₄ (3.37 mmol, 2 mol%) was added into the reactor and the condenser was replaced by a curved distillation column.

The mixture was allowed to stir at 140°C during 3h30 under vacuum (800 mbar) in order to remove water formed as a by-product during the esterification reaction.

After 3h30 at 140°C, NMR analysis showed a selectivity (monoester + bis-ester) of 83 mol% and the following approximate crude mixture composition: 80 mol% of bis-ester, 3 mol% of mono-ester, 2 mol% of esterified dimer and 4 mol% of ketone.

The mixture was then cooled down at room temperature (about 23°C) and 300 mL of toluene were added. The solution was transferred into a separating funnel and the organic phase was washed 3 times with 500 mL of an aqueous solution of NaOH (0.3M) to remove excess of chloroacetic acid. The organic phase was dried over MgSO₄, filtered and then evaporated to give 102.7 g of crude product.

The product could be easily purified by dissolving the oil in ethanol (the starting ketone being not soluble in ethanol) followed by a filtration over celite. The filtrate was evaporated to afford 94 g of black oil with a purity of 89 wt% for the bis-ester corresponding to a isolated yield of 80 % (RMN).

¹H NMR (CDCl₃, 400 MHz) δ (ppm): 5.11-5.02 (m, 2H), 4.04 (s, isomer 1, 2H), 4.03 (s, isomer 2, 2H), 1.66-1.49 (m, 4H), 1.43-1.19 (m, 50 H), 0.86 (t, J = 6.8 Hz , 6H).

¹³C NMR (CDCl₃, 101 MHz) δ (ppm): 167.14, 167, 76.22, 75.83, 40.92, 40.82, 31.96, 30.6, 29.72, 29.69, 29.63, 29.54, 29.39, 29.33, 29.28, 28.85, 25.47, 24.96, 22.72, 14.15 (terminal CH₃).

### Condensation with chloroacetic acid (with triflic acid as an optional catalyst)

The reaction was conducted under an inert argon atmosphere in a 500 mL three necked round bottom flask equipped with a magnetic stirrer, a heater, a condenser, a temperature probe and an insulated addition funnel. In the round bottom flask itself were added 85.63 g of chloroacetic acid (0.897 mol, 5 eq). In the insulated addition funnel were added 83.3 g of melted fatty epoxide (97 wt% purity, 0.179 mol, 1 eq).

The first step of monoester formation was conducted at 65°C without triflic acid to limit the formation of ketone and dehydration by-products. The fatty epoxide was therefore added drop-wise over 2h into the reactor containing chloroacetic acid at 65°C under stirring in order to limit the self-condensation of fatty epoxide. At the end of the addition, the mixture was left at 65°C under stirring during an additional hour. NMR analysis showed a conversion level >99 % for the starting epoxide.

For the formation of the bis-ester, 3.2 µL of 99% triflic acid (0.036 mmol, 0.02 mol%) were then added into the reaction mixture. The condenser was replaced by a curved distillation column and the mixture was allowed to stir at 140°C during 5h00 under a light vacuum (975 mbar) in order to remove water formed as a by-product of the esterification reaction.

After 5h00, NMR analysis showed a selectivity (monoester + bis-ester) of 88 mol% and a composition of 82 mol% of bis-ester, 6 mol% of mono-ester, 5 mol% of esterified dimer and 3 mol% of ketone.

The vacuum was then increased to 800 mbar and progressively until 10 mbar in order to distil chloroacetic acid, triflic acid catalyst and to complete conversion of mono-ester toward bis-ester.

Once all chloroacetic acid had been distilled out (verified by NMR analysis), the mixture was allowed to cool down at room temperature and atmospheric pressure was restored. The crude oil was transferred into a flask for purification. The product could be easily purified by dissolving the oil in ethanol (the starting ketone being not soluble in ethanol) followed by a filtration over celite. The filtrate was evaporated to afford 107.3 g of a black oil with a purity of 93 wt% for the bis-ester corresponding to an isolated yield of 89%.

¹H NMR (CDCl₃, 400 MHz) δ (ppm): 5.11-5.02 (m, 2H), 4.04 (s, isomer 1, 2H), 4.03 (s, isomer 2, 2H), 1.66-1.49 (m, 4H), 1.43-1.19 (m, 50 H), 0.86 (t, J = 6.8 Hz , 6H).

¹³C NMR (CDCl₃, 101 MHz) δ (ppm): 167.14, 167, 76.22, 75.83, 40.92, 40.82, 31.96, 30.6, 29.72, 29.69, 29.63, 29.54, 29.39, 29.33, 29.28, 28.85, 25.47, 24.96, 22.72, 14.15 (terminal CH₃)

### Quaternization reaction with NMe₃

The reaction was conducted under an inert argon atmosphere. In a 1L double-jacketed reactor equipped with a mechanical stirrer, a condenser and a temperature probe, were added:
- 107.3 g (93 wt% purity, 0.16 mol, 1 eq.) of chloroacetate bis-ester C₃₁
- 687 mL (1.28 mol, 8 eq.) of a solution of trimethylamine (NMe₃) in THF (∼ 2 mol/L) dried beforehand on molecular sieve activated the day before.

The reaction mixture was heated at 40°C and stirring was started at 1000 rpm. After 6h, NMR analysis in d₄-MeOH showed a conversion level >99 % for the starting bis-ester with a molar composition of ∼ 86 mol% for glycine betaine bis-ester. The reactor was drained, rinsed with CH₂Cl₂ and the volatiles were evaporated under vacuum.

The brown solid was reduced into a powder, deposited on a sinter filter and washed 5 times with 200 mL of ethyl acetate. The solid was dried under vacuum.

Then, the product was transferred into a 1L reactor equipped with a mechanical stirrer, a condenser, a heater and a temperature probe. The product was solubilized into 800 mL of chloroform and 150 g of activated charcoal pellets were added. The mixture was stirred at 40°C for 2 hours in order to whiten the product.

After 2h, the suspension was filtrated over celite and the solvent was evaporated to afford 109 g of a brown wax with a weight composition of ∼ 95 wt% of quaternary di-ammonium compound of formula (I), 2 wt% of quaternary mono-ammonium, 0.1 wt% of C₃₁ ketone and 4 wt% of the ether by-product. The purified yield of the glycine betaine bis-ester was ∼ 87%.

### Example 2 - Synthesis of a quaternary di-ammonium compound starting from C₁₆-C₁₈ (60:40) fatty acid cut

### Piria ketonization toward internal C₃₁-C₃₅ ketones cut

The reaction was conducted under an inert argon atmosphere in a 200 mL quartz reactor equipped with a heating mattress, a mechanical stirring (A320-type stirring mobile manufactured by 3D-printing with Inox SS316L), an insulated addition funnel, a distillation apparatus and a temperature probe.

In the reactor itself were introduced:
- 15 g of MASCID^{™} acid 1801 (from Musim Mas Group) with the following composition: 60.9 wt% of palmitic acid and 38.2 wt% of stearic acid (0.056 mole of fatty acids), and
- 1.15 g of MgO (0.028 mole).

In the insulated addition funnel were added 45 g of the same melted fatty acids mixture (0.167 mole).

The temperature of the reaction mixture was then raised to 250°C and once the temperature had reached 150°C, the stirring was started (1200 rpm). After 1h15 reaction time at 250°C, FTIR analysis showed complete conversion of the starting fatty acids into the intermediate magnesium carboxylate complex.

The temperature of the reaction mass was then raised further to 330°C and the mixture was allowed to stir at this temperature during 1h30 in order to allow decomposition of the magnesium complex to the desired ketone.

Then 15 g of the melted fatty acid mixture were progressively added to the reactor thanks to the addition funnel during 15 minutes and the mixture was stirred at 330°C during one additional hour. FTIR analysis confirmed complete conversion of fatty acids and magnesium complex to the desired ketone.

Two additional cycles of 15 g fatty acid addition during 15 minutes followed by one hour stirring at 330°C were then realized. Following the last addition cycle the mixture was allowed to stir at 330°C during one additional hour in order to ensure complete conversion of the intermediate magnesium carboxylate complex to the desired ketone, which was confirmed thanks to FTIR and NMR analysis according to the following protocol: the sample was dissolved in chloroform and washed three times with 2N HCI aqueous solution and the solvent was evaporated. NMR and IR analysis on the residue did not show any presence of starting fatty acid that might have been formed by complex hydrolysis.

The temperature of the reaction mixture was allowed to cool down at 60°C and the crude was solubilized in 350 mL of hot CHCl₃ (60°C). The suspension was filtered on a plug of silica (100 g) and the product was further eluted with additional amounts of CHCl₃. After solvent evaporation 50.5 g of crude product were obtained.

The product was washed 3 times with 200 mL of isopropanol on a sintered filter in order to remove trace of by-products. The solid was dried to afford the desired ketone as a white powder with a purity of 100 mol% corresponding to a yield of 90%.

### Hydrogenation of ketones mixture toward internal C₃₁-C₃₅ fatty alcohols mixture

Same protocol as the one described in example 1 under "Hydrogenation of 16-hentriacontanone to 16-hentriacontanol" part was followed to obtain the desired fatty alcohols mixture in excellent yield.

### Dehydration of C₃₁-C₃₅ fatty alcohols to internal olefins

The reaction was conducted under an inert argon atmosphere. In a 200 mL quartz reactor equipped with a heating mattress, a mechanical stirrer (A320-type stirring mobile manufactured by 3D-printing with Inox SS316L), surmounted by a condenser connected to a 50 mL two-necked distillate collection flask and a temperature probe were added:
- 43.6 g of the C₃₁₋₃₅ fatty alcohol (92 mmol, 1 eq)
- 4.4 g (43 mmol, 10 wt%) of Al₂O₃-η.

The reaction medium was first heated at 150°C in order to melt the fatty alcohol mixture and stirring was started (about 500 rpm). Then, the temperature was increased up to 300°C and the mixture was allowed to stir at 1000 rpm under argon atmosphere. The reaction progress was monitored thanks to NMR analysis. After 2 hours reaction time at 300°C, NMR analysis in CDCl₃ showed complete conversion of the fatty alcohol and also presence of 0.3 mol% of ketone which had been formed as a by-product.

Stirring and heating were then stopped to allow the crude media to cool down. Once the temperature had decreased to about 80°C the molten crude was transferred to a beaker. The reactor vessel and the stirring mobile were rinsed with chloroform (Al₂O₃ is insoluble).

The suspension was filtered to remove catalyst and the solvent was evaporated under vacuum to afford 40.1 g of a clear yellow oil which solidified at room temperature to give a white compact solid in the form of a wax (>99 wt% purity) and 95% yield (NMR).

### Epoxidation of internal olefins to afford C₃₁₋₃₅ oxiranes

The reaction was conducted under an inert argon atmosphere. In a 300 mL double-jacketed reactor equipped with a mechanical stirrer (propeller with four inclined plows) and baffles, a condenser, an addition funnel and a temperature probe were added:
- 39 g of C₃₁₋₃₅ alkene (>99 wt% purity, 86 mmol)
- 7.3 mL (7.7 g, 128 mmol) of acetic acid, and
- 11.7 g (30 wt%) of Amberlite^{®} IR 120H resin.

The mixture was heated to 75°C in order to melt the fatty alkene. Stirring was then started and 13.1 mL (14.6 g, 128 mmoles) of H₂O₂ 30% were slowly added into the mixture using the addition funnel while monitoring temperature of the reaction medium in order to avoid temperature increase of the reaction mass (slight exothermicity). This required about 20 min. During the addition, stirring rate was increased to improve mass transfers due to the heterogeneous nature of the reaction media. At the end of the addition, the temperature of the reaction medium was increased to 85°C. After 4h30 of stirring, NMR analysis showed an alkene conversion level around 97%.

In order to complete the reaction, additional 4.4 mL (4.8 g, 43 mmol) of H₂O₂ 30% were added to the reaction media. After 7 hours of total reaction time at 85°C, NMR analysis showed a complete conversion level for the alkene.

Stirring was then stopped in order to allow the liquid phases to separate in the reactor at 85°C. Solid Amberlite^{®} resin had sedimented at the bottom of the reactor. Aqueous phase was removed by draining through the bottom valve. 100 mL of water were then added into the reactor and the mixture was allowed to stir during 15 minutes. The phases were again separated and the aqueous phase was removed.

Finally, the melted organic phase was drained into a PYREX^{®} beaker (with the catalyst) and the reactor was rinsed 2 times with 100 mL of chloroform in order to recover remaining product. The organic phase was dried over MgSO₄, filtered and evaporated to afford 40.1 g of a white compact solid with a purity of 99 wt% (epoxide + di-alcohol by-product). The yield taking into account the purity was 98%.

### Catalyst-free condensation with chloroacetic acid to afford chloroacetate bis-ester C₃₁₋₃₅

The reaction was conducted under an inert argon atmosphere in a 500 mL three necked round bottom flask reactor equipped with a magnetic stirrer, a heater, a condenser, a temperature probe and an insulated addition funnel. In the bottom flask reactor itself were added 39.19 g of chloroacetic acid (410.5 mmol, 5 eq). In the insulated addition funnel maintained at 65°C were added 38.6 g of melted fatty epoxide (purity: 99 wt%, 82.1 mmol, 1 eq).

The first step of hydroxyl-ester formation was conducted at 65°C to limit the formation of ketone and dehydration by-products. The fatty epoxide was therefore added drop-wise over 1h30 into the reactor containing chloroacetic acid at 65°C under stirring in order to limit the self-condensation of two fatty epoxide molecules. Upon completion of the addition of the fatty epoxide, the mixture was allowed to stir at 65°C during 30 min. NMR analysis showed a conversion level >98 % for the starting epoxide.

For the formation of the final bis-ester, the condenser was replaced by a curved distillation column and the mixture was allowed to stir at 140°C during 5h30 under a light vacuum (975 mbar) in order to assist the removal of water which was formed as a by-product of the esterification reaction. After 5h at 140°C, NMR analysis showed a selectivity (monoester + bis-ester) of 92 mol% and the following approximate crude mixture composition: 90 mol% of bis-ester, 2 mol% of mono-ester, 1 mol% of esterified dimer and 2 mol% of ketone.

The pressure was then decreased down to 800 mbar and progressively until 10 mbar in order to distil the excess of chloroacetic acid and to complete conversion of mono-ester toward bis-ester. Once the whole amount of chloroacetic acid had been distilled out (verified by NMR analysis), the mixture was cooled down to room temperature and the atmospheric pressure was re-established. The crude oil was then transferred into a flask for purification.

The product could be easily purified by dissolving the oil in 300 mL of isopropanol (the starting ketone being not soluble in isopropanol) followed by a filtration over celite. The filtrate was evaporated to afford 49.1 g of a black oil with a purity of 93 wt% for the bis-ester corresponding to an isolated yield of 86 % (RMN).

¹H NMR (CDCl₃, 400 MHz) δ (ppm): 5.11-5.02 (m, 2H), 4.04 (s, isomer 1, 2H), 4.03 (s, isomer 2, 2H), 1.66-1.49 (m, 4H), 1.43-1.19 (m, 52.8 H (average number)), 0.86 (t, J = 6.8 Hz , 6H).

¹³C NMR (CDCl₃, 101 MHz) δ (ppm): 167.14, 167, 76.22, 75.83, 40.92, 40.82, 31.96, 30.6, 29.73, 29.64, 29.54, 29.39, 29.28, 28.85, 25.47, 24.96, 22.72, 14.15 (terminal CH₃).

### Quaternization with NMe₃

The reaction was conducted under an inert argon atmosphere. In a 1L double-jacketed reactor equipped with a mechanical stirrer, a condenser and a temperature probe, were added:
- 48.1 g (69 mmol, 1 eq) of chloroacetate bis-ester C₃₁₋₃₅ (purity: 93 wt%)
- 334 mL (625 mmol, 9 eq) of a solution of trimethylamine in THF (∼ 2 mol/L).

The reaction mixture was then stirred (1000 rpm) at 40°C. After 3h, the mixture was allowed to cool down at room temperature and stirred overnight. The next day, NMR analysis (d₄-MeOD) showed a full conversion of the starting bis-ester with an approximate selectivity of 92 mol% (NMR) toward the desired product glycine betaine bis-ester. The reactor was drained, rinsed with THF and the volatiles were removed under vacuum.

The product was reduced to a powder, deposited on a sinter filter and washed 5 times with 100 mL of ethyl acetate in order to remove the organic impurities. The solid was dried under vacuum to afford 53 g of a brown wax with the following approximate weight composition: 94 wt% of NQ19 quaternary bis-ammonium, 2 wt% of quaternary mono-ammonium, 0.1 wt% of N(Me)₃.HCl and 3.5 wt% of an ether by-products. The purified yield of the glycine betaine bis-ester was 94%.

### Example 3 - Synthesis of a quaternary ammonium compound corresponding to formula (I) starting from C₃₁ 16-hentriacontanone

C₃₁ internal olefin was obtained from palmitic acid following Piria ketonization, according to the protocol described in US patent 10035746, chain of examples 2 to 4.

### Epoxidation of internal olefin to fatty epoxide

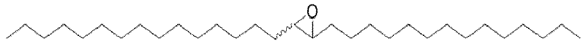

The reaction was conducted under an inert argon atmosphere.

In a 1L double-jacketed reactor equipped with a mechanical stirrer (propeller with four inclined plows), a condenser, an addition funnel and a temperature probe were added 61.9 g of C₃₁ alkene (0.142 mol), followed by 16.3 mL (17.1 g, 0.285 mol) of acetic acid and 13.6 g (22 wt%) of Amberlite^{®} IR 120H resin. The mixture was heated to 65 °C to melt the fatty alkene. The agitation was started and then 21.8 mL (24.2 g, 0.214 mol) of an aqueous solution of H₂O₂ (conc. 30%) was slowly added to the mixture using the addition funnel at a rate avoiding a significant temperature increase. This required about one hour. The temperature was then increased to 75°C and the reaction mixture was allowed to stir overnight (after 15 min, NMR analysis showed that the conversion level was already around 60% with 99% selectivity). Then additional 10.2 mL (11.3 g, 0.1 mol) of an aqueous solution of H₂O₂ (30%) was added slowly and after 4 hours following the second addition of H₂O₂ NMR analysis showed that the conversion level was around 88% (98% selectivity). Another addition of 8.14 mL of acetic acid (8.55 g, 0.142 mol) followed by 11.6 mL of 30% H₂O₂ (12.91 g, 0.114 mol) was finally performed in order to increase the conversion level.

The mixture was allowed to stir a second night at 75°C.

Finally NMR analysis showed a conversion level of 93% (95% selectivity).

The mixture was allowed to cool down to room temperature and then 300 mL of chloroform were added. The mixture was transferred to a separating funnel and the organic phase was washed three times with 300 mL of water and then the aqueous phase was extracted twice with 100 mL of chloroform. The Amberlite^{®} solid catalyst stayed in the aqueous phase and was removed during the first separation with the aqueous phase. The organic phases were collected, dried over MgSO₄, filtered and evaporated to give 65.3 g of a white solid with a purity of 91% w/w (epoxide + di-alcohol).

The yield taking into account the purity was 92%.

### Hydrolysis of fatty epoxide to afford fatty diol

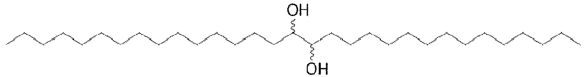

The reaction was conducted under an inert argon atmosphere.

In a 1L double-jacketed reactor equipped with a mechanical stirrer (propeller with four inclined plows), a condenser and a temperature probe were added 82.9 g of C₃₁ epoxide (purity: 94.5 wt%, 0.174 mol) followed by 480 mL of methyl-THF.

The mixture was allowed to stir at room temperature and 73 mL of a 3 M aqueous solution of H₂SO₄ was then added. The reaction medium was then stirred at 80°C during 90 minutes. NMR analysis showed that the reaction was completed. The biphasic mixture was allowed to cool down to room temperature and the organic phase was separated. The solvent was then removed under vacuum and the residue was suspended in 200 mL of diethyl ether. The suspension was filtered and the resulting solid was washed 3 times with 50 mL of diethyl ether. The white solid was finally washed 2 times with 50 mL of methanol and was dried under vacuum to remove traces of solvent.

At the end 75.53 g of product was obtained as a white powder with a purity of 95.7 % w/w corresponding to a yield of 89 %.

### Esterification and quaternization of fatty diol with trimethylglycine to afford compound of formula (I)

All the reactions were conducted in carefully dried vessels and under an inert argon atmosphere. Fresh commercial anhydrous CHCl₃ (amylene stabilized) and anhydrous toluene were used as such. Betaine hydrochloride (19.66 g, 128.4 mmoles) was washed ten times with 20 mL of anhydrous THF followed by drying under vacuum to remove traces of solvent prior to use.

In a 100 mL four-neck round-bottom flask equipped with a magnetic stirrer, a heater, a condenser, a temperature probe and a curved distillation column connected to two traps of NaOH were quickly added:
19.66 g of dried betaine hydrochloride (128.4 mmoles) and
28 mL of SOCI2 (45.86 g, 0.386 mol). The heterogeneous mixture was stirred and the temperature was then slowly increased to 70°C. It was observed that when the temperature reached 68°C, gas was released (SO₂ and HCI) and the mixture turned homogeneous yellow.

The mixture was then allowed to stir at 70°C during two hours and hot anhydrous toluene (25 mL, 80°C) was added into the vessel. The mixture was stirred and then decanted at 0°C (white-yellow precipitate formation) and the upper phase of toluene was removed through a cannula. The operation of toluene washing was repeated seven times in order to remove all SOCl₂ excess. NMR analysis showed complete conversion of glycine betaine hydrochloride but also formation of NMe₃·HCl adduct (NMe₃HCl content in the solid: 12.3 mol%).

20 mL of dry CHCl₃ was then added to the solid betainyl chloride.

A solution of 26.19 g (56 mmol) of fatty diol in 90 mL of anhydrous CHCl₃ was prepared at 55°C and was added dropwise under stirring to the reaction vessel at room temperature (exothermicity and emission of HCI was observed). The mixture was then allowed to stir at 55°C overnight. Over the course of the reaction, the mixture turned homogeneously orange. NMR analysis showed that the conversion level was around 100%.

The mixture was then allowed to cool down to room temperature and the solvent was evaporated under vacuum. The residue was solubilized in methanol at 0°C and the formed precipitate was filtered out. The obtained filtrate was then evaporated to give 39.7 g of crude product. This product was then deposited on a sinter filter and washed with cyclohexane to remove some remaining organic impurities. The resulting washed solid was dried under vacuum to afford 22 g of crude material. A final purification with a mixture of CH₂Cl₂/cyclohexane 50:50 was carried out; the solid was solubilized again in this solvent mixture at 50°C and was allowed to cool down to room temperature. The formed precipitate was filtered out and after evaporation of the filtrate 19 g of a beige wax was obtained with the following composition:
95 wt% of glycine betaine diester
1.5 wt% of methyl betainate
2 wt% of trimethylamine hydrochloride
1.5 wt% of glycine betaine hydrochloride.

The purified yield was 44%.

### Example 4 - Evaluation of adsorption properties on nanocellulose crystals

Adsorption of cationic surfactant on negatively charged surface is an important property for various applications. This property is linked to the minimal concentration of cationic surfactant needed to produce aggregation of negatively charged cellulose nano crystal (CNC) in suspension in aqueous media. Comparison of the aggregate size can be monitored by dynamic light scattering (DLS).

Following the protocol described in literature (Ref.: E. K. Oikonomou, et al., J. Phys. Chem. B, 2017, 121 (10), pp 2299-2307), adsorption properties of quaternary ammonium were investigated by monitoring the ratio X=[surfactant]/[CNC] or the mass fraction M = [surfactant]/([surfactant]+[CNC]), at fixed [surfactant]+[CNC]= 0.01wt% in aqueous solution, required to induce the agglomeration of the cellulose nano crystal.

The range of CNC aggregation correspond to the range of ratio X (or M) triggering an aggregation of CNC, i.e. the range where the aggregate size measured by DLS is higher than a pure aqueous solution of CNC or an aqueous solution of surfactant at 0.01wt%.

Ranges of X and M of aggregation of CNC are summarized in Table 1. The lower range of aggregation X or M, the better the adsorption properties on negatively charged surface

**Table 1**

| | Range of CNC aggregation (ratio) X=[surfactant]/[CNC] Xₘᵢₙ - Xₘₐₓ | Range of CNC aggregation (mass fraction) Mₘᵢₙ - Mₘₐₓ |
|---|---|---|
| Compound of example 3 | 0.1-1.82 | 0.09-0.65 |
| Fentacare^{®} TEP | 1-33 | 0.50-0.97 |

Fentacare^{®} TEP was used as a comparison. Fentacare^{®} TEP is a commercial surfactant representing the benchmark.

The data show that the surfactant properties of the compound in accordance with the present invention is superior compared to the commercial surfactant Fentacare^{®} TEP.

### Example 5 - Determination of biodegradability

Biodegradability of the test substances has been measured according to the 301 F OECD protocol.

A measured volume of inoculated mineral medium, containing a known concentration of the test substance in order to reach about 50 to 100 mg ThOD/I (Theoretical Oxygen Demand) as the nominal sole source of organic carbon, was stirred in a closed flask (Oxitop^{™}respirometric flask) at a constant temperature (20 ± 2°C) for up to 28 days. Oxitop^{™} respirometric bottles were used in this test in order to access the biodegradability of the test samples: sealed culture BOD flasks were used at a temperature of 20±2 C during 28 days.

Evolved carbon dioxide was absorbed by pellets of Natrium or Potassium hydroxide present in the head space of the bottle. The amount of oxygen taken up by the microbial population (= oxygen consumption expressed in mg/l) during biodegradation process (biological oxidation of the test substance) decreased the pressure of the head space (Δ P measured by the pressure switch) and was mathematically converted in mg O₂ consumed /litre. Inoculum corresponded to a municipal activated sludge washed in mineral medium (ZW media) in order to decrease the DOC (Dissolved Oxygen Carbon) content. Control solutions containing the reference substance sodium acetate and also toxicity control (test substance + reference substance) were used for validation purposes. Reference substance, sodium acetate, has been tested in one bottle (at a nominal concentration of 129 mg/l corresponding to 100 mg ThOD/I) in order to check the viability of the inoculum. Toxicity control corresponds to the mixture of the substance reference and the test substance; it will check if the test substance is toxic towards the inoculum (if so, the test has to be redone at a lower test substance concentration, if feasible regarding the sensitivity of the method).

As the substances of the present invention are for a majority of them not very soluble in water (if some are soluble in water, their metabolite after hydrolysis containing the alkyl chain has often very low solubility in water), we used a specific protocol named the "emulsion protocol". This protocol enabled us to increase the bioavailability of the poorly water soluble substance in the aqueous phase where we had the inoculum.

Emulsion protocol consisted of adding the test substance in the bottle through a stock solution made in an emulsion.

Emulsion was a 50/50 v/v mixture of a stock solution of the test substance dissolved in a non-biodegradable surfactant (Synperonic^{®} PE 105 at 1 g/l) and then mixed with a mineral silicone oil AR 20 (Sigma).

The first dissolution of the test substance in the non-biodegradable surfactant solution often required magnetic stirrer agitation followed by ultrasonication.

Once the dissolution was made, we mixed the aqueous solution with a mineral silicone oil at a 50/50 volume/volume ratio. This emulsion was maintained by magnetic stirrer agitation and was sampled for an addition in the corresponding bottle in order to reach the required test substance concentration.

Two emulsion controls were run in parallel during the test in order to remove their value from the emulsion bottle containing the test substance added through the emulsion stock solution.

The result of the biodegradability test is provided in Table 2 here below:

**Table 2**

| | Biodegradability after 28 days |
|---|---|
| Compound of example 3 | 92 % (OECD 301F) |

The result shows that the compound of example 3 has outstanding biodegradability. This beneficial effect is achieved without detrimentally affecting the surfactant properties of the compounds.

The ionic compounds of the present invention show a good combination of surfactant properties combined with a good biodegradabilty, a combination which is in many cases not achieved by commercial surfactants.

Since the ionic compounds of the present invention are also easily available starting from internal ketones which are easily accessible from fatty acids or fatty acid derivatives, the compounds of the present invention also provide economical benefits over the prior art compounds.

## Claims

1. A method for obtaining a diester having the general formula (IV) 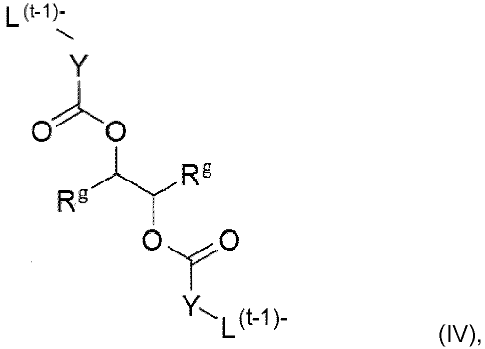 said method comprising reacting an epoxide of formula (V) 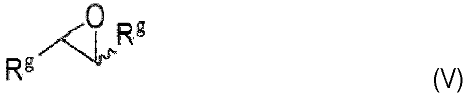 with a carboxylic acid of general formula (VI)
[L-Y-CO₂H]^{(t-1)-}[U^{u+}]_{(t-)/u} (VI)
wherein, wherever used in above formulae :
- R^{g}, which may be the same or different at each occurrence, is an aliphatic or halogenophatic group ;
- Y is a divalent C₁-C₆ aliphatic radical ;
- L is a leaving group ;
- t is an integer which is equal to 1 or which is equal or superior to 2 ;
- U^{u+} is a cation, and
- u is an integer fixing the positive charge of the cation.

2. The method according to claim 1 wherein the reaction proceeds with the elimination of one mole of water by one mole of epoxide, as represented by: 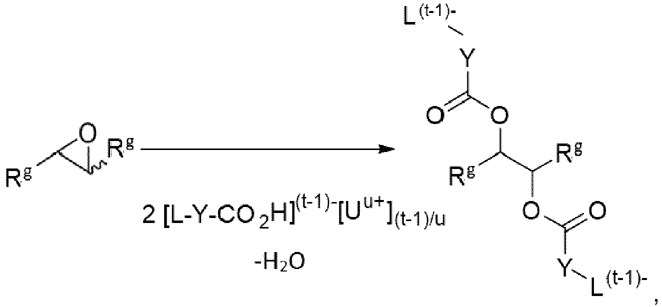 preferably in a reaction medium from which water is removed by distillation over the course of the reaction.

3. The method according to claim 1 or 2 wherein the reaction is conducted in the absence of enzyme.

4. The method according to any one of claims 1 to 3 wherein the reaction is conducted in the absence of any catalyst.

5. The method according to any one of claims 1 to 4 wherein the reaction is conducted under an inert atmosphere, such as a nitrogen or rare gas atomosphere.

6. The method according to any one of claims 1 to 5 wherein the total number of moles of carboxylic acid of formula (VI) which is contacted with the epoxide during the whole course of the reaction is at least four times higher than the number of moles of epoxide.

7. The method according to any one of claims 1 to 6 wherein the reaction takes place in a reactor where the epoxide and the carboxylic acid of formula (VI) are in molten state.

8. The method according to any one of claims 1 to 7 wherein the epoxide is added progressively, preferably continuously, in a reactor containing the whole amount of the carboxylic acid of formula (VI).

9. The method according to any one of claims 1 to 8 which comprises:
- a first step S₁ wherein the epoxide is reacted with the carboxylic acid of formula (VI) at a temperature T₁ below 100°C for a time t₁ which is sufficient to convert more than f₁=80 mol.% of the epoxide into a monohydroxyl-monoester of formula (VII): 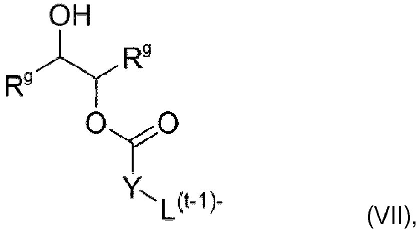
- a second step S₂ wherein the monohydroxyl-monoester and the epoxide which has not been converted at step S₁ into the monohydroxyl-monoester are reacted with the carboxylic acid of formula (VI) at a temperature T₂ of at least 100°C for a time t₂ which is sufficient to form the diester of formula (IV) in a molar amount greater than f₂=50 mol.% of the total amount of epoxide which is reacted with the carboxylic acid.

10. The method according to claim 9 wherein the whole amount of the epoxide is added progressively, preferably continuously, during step S₁ in a reactor containing the whole amount of the carboxylic acid of formula (VI).

11. The method according to claim 9 or 10 wherein
- T₁ ranges from 40°C to 80°C, t₁ ranges from 30 min to 3 h and f₁ is 90 mol.%, preferably 95 mol.%, more preferably 98 mol.%, and
- T₂ ranges from 120°C to 170°C, t₂ ranges from 3 h to 7 h and f₂ is 65 mol. %, preferably 75 mol.%, more preferably 80 mol. %.

12. The method according to any one of claims 9 to 11 wherein step S₂ is conducted at a pressure P₂ from 95 kPa to 99 kPa.

13. The method according to claim 12 which comprises, upon completion of step S₂, reducing progressively the pressure P₂ to a pressure P₃ of at most 20 kPa.

14. The method according to any one of claims 1 to 13 wherein R^{g} represents a C₅-C₂₇ aliphatic group.

15. A monohydroxyl-monoester compound of formula (VII) 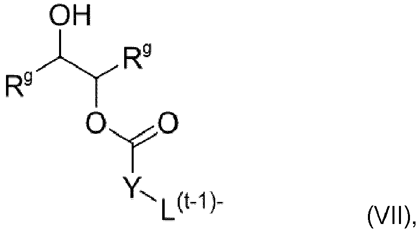 wherein
- R^{g}, which may be the same or different at each occurrence, represents a C₅-C₂₇ aliphatic group, preferably an alkyl group with 10 to 20 carbon atoms,
- Y is a divalent C₁-C₆ aliphatic radical,
- L is a leaving group, and
- t is an integer which is equal to 1 or which is equal or superior to 2.
